(19) Europäisches Patentamt
European Patent Office
Office européen des brevets

(11) EP 0 963 998 B1

(12) EUROPEAN PATENT SPECIFICATION

(45) Date of publication and mention of the grant of the patent:
**06.05.2004 Bulletin 2004/19**

(21) Application number: **98905646.0**

(22) Date of filing: **26.02.1998**

(51) Int Cl.[7]: **A61K 31/58**, A61P 35/00

(86) International application number:
**PCT/JP1998/000767**

(87) International publication number:
**WO 1998/038207 (03.09.1998 Gazette 1998/35)**

# (54) AZASTEROID COMPOUND FOR TREATING OR PREVENTING PROSTATIC CANCER

AZASTEROIDVERBINDUNG ZUR BEHANDLUNG ODER VORBEUGUNG VON PROSTATAKREBS

COMPOSE AZASTEROIDE POUR LE TRAITEMENT OU LA PREVENTION DU CANCER DE LA PROSTATE

(84) Designated Contracting States:
**AT BE CH DE DK ES FI FR GB GR IE IT LI LU MC NL PT SE**

(30) Priority: **26.02.1997 JP 4233597**

(43) Date of publication of application:
**15.12.1999 Bulletin 1999/50**

(73) Proprietor: **Sankyo Company Limited**
**Chuo-ku, Tokyo 103-8426 (JP)**

(72) Inventors:
- **KOJIMA, Koichi, Sankyo Company, Limited Tokyo 140-8710 (JP)**
- **KOBAYASHI, Tomowo, Sankyo Company, Limited Tokyo 140-8710 (JP)**

(74) Representative: **Gibson, Christian John Robert**
**MARKS & CLERK,**
**57/60 Lincoln's Inn Fields**
**London WC2A 3LS (GB)**

(56) References cited:
**EP-A- 0 484 094**
**EP-A- 0 923 942**
**EP-A- 0 998 930**
**JP-A- 5 032 693**
**JP-A- 8 073 492**
**JP-A- 61 257 996**
**US-A- 4 732 897**

Remarks:
The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

[Technical Field]

The present invention relates to use of a compound for producing a pharmaceutical preparation for treatment or prevention of prostate cancer.

[Background Art]

Testosterone 5α reductase inhibitor is known as a therapeutic agent for benign prostatic hypertrophy that reduces prostate size by a mechanism of action that inhibits prostate cell growth caused by excessive male hormones (and mainly dihydrotestosterone). In addition, testosterone 5α reductase inhibitors such as Finasteride are sold as a therapeutic agent for benign prostatic hypertrophy in the United States of America and Europe. There are some testosterone 5α reductase inhibitors on which clinical studies are being conducted in order to develop them as therapeutic agents for prostate cancer.
The reductive effects on the prostate correspond to the potency of testosterone 5α reductase inhibitory activity. Different from benign prostatic hypertrophy, however, therapeutic effects on prostate cancer do not correspond to the potency of testosterone 5α reductase inhibitory activity alone, since a greater number of factors are involved with respect to proliferation of prostate cancer.
Japanese Patent Application (Kokai) No. Hei 8-73492 discloses a number of azasteroid compounds which have testosterone 5α-reductase inhibitory effects.
The compound used in the present invention has also been used in JP Hei 5-032693, EP 0 484 094, and EP 0 998 930.

[Disclosure of the Invention]

The inventors of the present invention conducted an earnest research on the synthesis of derivatives having testosterone 5α reductase inhibitory activity and their pharmacological activity over many years. The inventors found that the compound used in the present invention has an excellent prostate cancer therapeutic or preventive effect and accomplished the present invention.
The object of the present invention is to provide a use of the above-mentioned compound for the manufacture of a pharmaceutical for treatment or prevention of prostate cancer.
Namely, the composition for treating or preventing prostate cancer used in the present invention contains as its active ingredient N-[1-methyl-1-(4-methoxyphenyl)ethyl]-3-oxo-4-aza-5α-androst-1-ene-17β-carboxamide or a pharmacologically acceptable salt thereof, or another derivative thereof, and preferably is taken orally.
Thus, the present invention provides the use of N-[1-methyl-1-(4-methoxyphenyl)ethyl]-3-oxo-4-aza-5α-androst-1-ene-17β-carboxamide, pharmacologically acceptable salts thereof or another derivative thereof for producing a pharmaceutical for treatment or prevention of prostate cancer, and preferably it is taken orally.
The term "pharmacologically acceptable salts thereof" means the salts of the compound used in the present invention, which can be converted to salts thereof, examples of such salts preferably include alkali metal salts such as a sodium salt, a potassium salt and a lithium salt, alkaline earth metal salts such as a calcium salt and a magnesium salt, and metal salts such as an aluminum salt, an iron salt and a zinc salt.
Further, the compound used in the present invention, when it is allowed to stand in the atmosphere, may absorb some moisture, and it may, as a result, be associated with absorption water or it may be converted to a corresponding hydrate. Such compounds are also included in the present invention.
The compound used in the present invention can be prepared according to the process shown below.

[Process A]

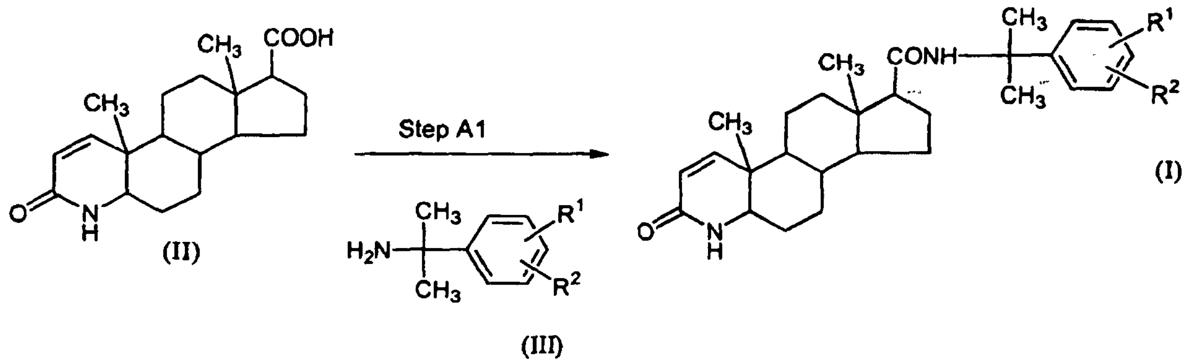


wherein $R^1$ represents H and $R^2$ represents a para-methoxy group.

Process A is a method for preparing the desired compound (I) by condensing a carboxylic acid derivative (II) with an amine derivative (III).

Step A1 is to prepare the compound (I) by a reaction of compound (II) or a reactive derivative thereof with a compound (III). The reaction is carried out according to the conventional methods in peptide synthesis, for example an azide method, an active ester method, a mixed acid anhydride method or a condensation method.

In the above methods, the azide method is carried out as follows: the compound (II) or an ester thereof is reacted with hydrazine in an inert solvent (for example, dimethylformamide) at approximately room temperature to prepare an amino acid hydrazide. The amino acid hydrazide is reacted with a nitrous acid compound to afford an azide derivative, followed by the treatment of the azide derivative with an amine derivative (III).

The nitrous acid compound employable here may include, for example, alkali metal nitrites such as sodium nitrite or alkyl nitrites such as isoamyl nitrite.

The reaction is preferably carried out in an inert solvent, and the solvent employable here may include, for example, amides such as dimethylformamide and dimethylacetamide, sulfoxides such as dimethyl sulfoxide and pyrrolidones such as N-methylpyrrolidone. Two step reactions [preparation of azide and amide derivative (I)] are generally carried out in one reaction pot. The reaction temperature is -50°C to 0°C for the former reaction and -10°C to 10°C for the latter reaction, and the reaction time is 5 minutes to 1 hour for the former reaction and 10 hours to 5 days for the latter reaction.

The active ester method is carried out by a reaction of the compound (II) with an active esterification agent to give an active ester, followed by reaction of the active ester with an amine derivative (III).

Both reactions are preferably carried out in an inert solvent, and the solvent employable here may include, for example, halogenated hydrocarbons such as methylene chloride and chloroform, ethers such as diethylether and tetrahydrofuran, amides such as dimethylformamide and dimethylacetamide, and nitriles such as acetonitrile.

The active esterification agent employable here may include, for example, N-hydroxyl compounds such as N-hydroxysuccinimide, 1-hydroxybenzotriazole and N-hydroxy-5-norbornene-2,3-dicarboximide or disulfide compounds such as dipyridyldisulfide, and the active esterification reaction is preferably carried out in the presence of a condensation agent such as dicyclohexylcarbodiimide, carbonyldiimidazole or triphenylphosphine.

The reaction temperature is -10°C to 100°C in the active esterification reaction and approximately room temperature in the reaction of the active ester compound with the amine derivative (III), and the reaction time is 30 minutes to 80 hours for both reactions.

In the reaction of the active ester with the amine, 4-dimethylaminopyridine may be added to the reaction system.

The mixed acid anhydride method is carried out by preparing a mixed acid anhydride of the compound (II), followed by reaction of the mixed acid anhydride with an amine derivative.

The reaction for preparing the mixed acid anhydride derivative is accomplished by a reaction of the compound (II) with an agent for forming a mixed acid anhydride derivative [for example, lower ($C_1$-$C_4$) alkyl halogenated carbonic acids such as ethyl chlorocarbonate and isobutyl chlorocarbonate, lower alkanoyl halides such as pivaloyl chloride, lower alkyl or diaryl cyanophosphoric acids such as diethyl cyanophosphate and diphenyl cyanophosphate, or sulfonyl halides such as 2,4,6-triisopropylbenzenesulfonyl chloride, paratoluenesulfonyl chloride and methanesulfonyl chloride] in an inert solvent (for example, the halogenated hydrocarbons, amides and ethers described above). The reaction is

preferably carried out in the presence of organic amines such as triethylamine and N-methyl morpholine, and the reaction temperature is -10°C to 50°C and the reaction time is 30 minutes to 20 hours.

The reaction of the mixed acid anhydride derivative with the amine derivative (III) is preferably carried out in an inert solvent (for example, the halogenated hydrocarbons, amides and ethers described above) in the presence of the organic amines. The reaction temperature is 0°C to 80°C and the time required for the reaction is 1 hour to 48 hours.

The reaction is carried out in the coexistence of the compound (II), the compound (III) and an agent for forming a mixed acid anhydride derivative without isolation of a mixed acid anhydride derivative.

The condensation method is carried out by a reaction of the compound (II) with the amine derivative (III) directly in the presence of a condensation agent such as dicyclohexylcarbodiimide, carbonyldiimidazole or 2-chloro-1-methylpyridinium iodide/triethylamine. The present reaction is carried out in a similar manner to that described in the preparation of the active ester.

In the case where a protected hydroxyl group is present in $R^1$ and $R^2$, the protecting group can be removed according to conventional methods.

The raw material compound (II) or the active ester thereof is known or is prepared according to known methods [for example, J. Med. Chem., 27, 1690 (1984); J. Med. Chem., 29, 2298 (1986)].

Further, the compound (III) is known or is prepared according to known methods [for example:

Synthesis, 593 (1976);

J. Org. Chem., 36, 305 (1971);

Angew. Chem., 82, 138 (1970);

Synthesis, 24 (1978);

Synthetic Commun., 18, 777 (1988);

Synthetic Commun., 18, 783 (1988);

Organic Reaction, 3, 337 (1946);

Org. Synthesis, 51, 48 (1971);

Tetrahedron. 30, 2151 (1974); and

J. Org. Chem., 37, 188 (1972)], and, for example, a raw material compound of the present invention having a $H_2N$-C(Me)(Me)-Ph($R^1$)($R^2$) moiety is prepared according to the method described in Synthesis, P. 24 (1978). The reaction scheme is shown below:

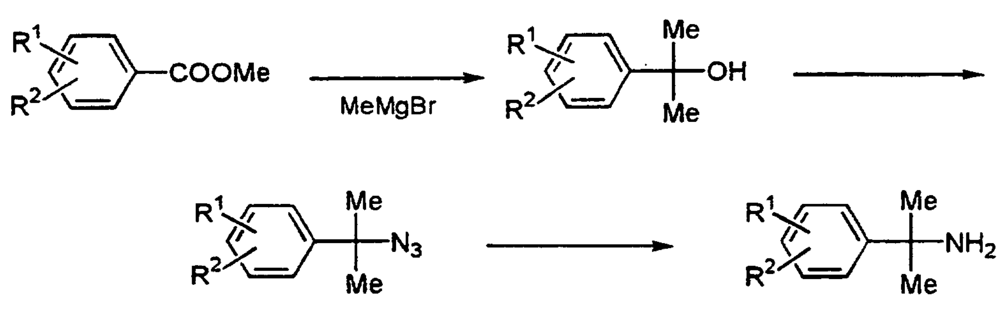


(wherein $R^1$ and $R^2$ have the same meanings as defined above, Me represents a methyl group and Ph represents a phenyl group), and comprises a Grignard reaction, an azidation reaction of hydroxyl group and a reduction reaction.

[Brief Description of Figure]

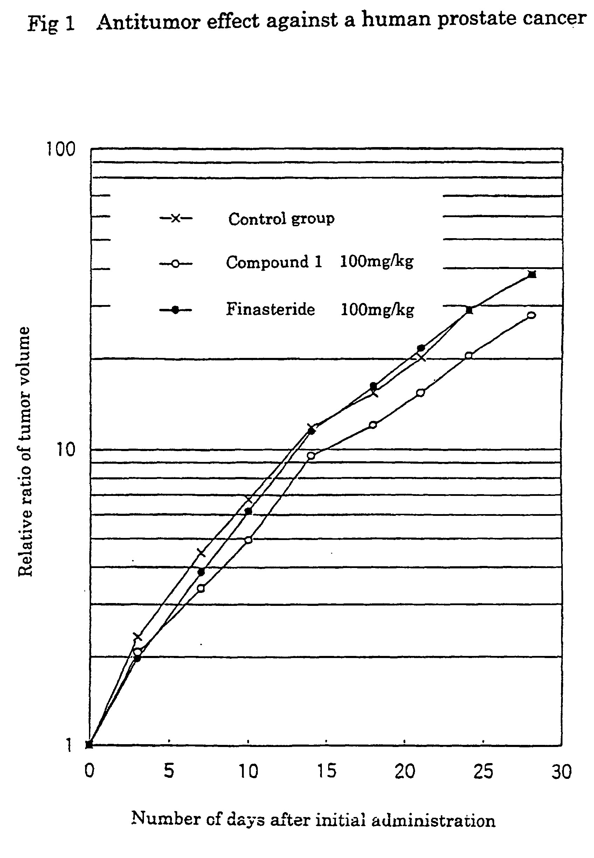

Fig. 1 shows an antitumor effect against a human prostate cancer in Example 1.

Vertical axis: Relative ratio of tumor volume (expressed in logarithm)

Horizontal axis: Number of days after initial administration

cross dots : Control group

Black dots : Finasteride administration group

circlet dots: Compound 1 administration group

Compound 1; N-[1-methyl-1-(4-methoxyphenyl)ethyl]-3-oxo-4-aza-5α-androst-1-ene-17β-carboxamide

[Best mode for carrying out the invention]

In the following, the present invention will be described in further detail with reference to Examples and Reference Example, but the scope of the present invention is not limited thereto.

Example 1 Antitumor Activity Test 1

A solid tumor, which was reliably taken beneath the skin was obtained by subculturing subcutaneously human prostate cancer strain LNCaP of a cultured cell system purchased from the American Type Culture Collection (ATCC) five to six times in male and female nude mice. This human prostate cancer strain was used in the test. A solid cancer fragment of this strain 3 mm square was transplanted beneath the skin of the axillary region of BALB/cA Jc1-nu nude mice (Nippon Clea, males, 8 weeks old). Mice in which the tumor was reliably taken about 20 days later were randomly assigned to groups of 8-10 animals each. N-[1-methyl-1-(4-methoxyphenyl)ethyl]-3-oxo-4-aza-5α-androst-1-ene-17β-carboxamide (compound 1, 100 mg/kg) or Finasteride (100 mg/kg) were orally administered to the mice at the rate of 0.1 ml/10 g of body weight once a day in the morning for 28 consecutive days. Tumor diameter was measured twice a week, and tumor volume V ($V = ab^2/2$) was calculated from the major axis (a) and minor axis (b). The relative volume ratio for each mouse was represented as Vn/Vo. The tumor growth inhibition rate (%) was determined from the relative volume ratio for each mouse.

$$\text{Tumor growth inhibition rate (\%)} = (1 - Vn/Vo) \times 100$$

Vn: Tumor volume on nth day

Vo: Tumor volume on first day of administration

Results

Relative volume ratio data is shown in Fig. 1.
Finasteride is sold in the United States of America and Europe as a drug for the treatment of benign prostatic hypertrophy, and clinical studies for its use as a drug for the treatment of prostate cancer are currently being conducted.
Fig. 1 shows that Finasteride does not exhibit any tumor growth inhibitory activity whatsoever, but the compound 1, however, exhibits tumor growth inhibitory activity of which the maximum tumor inhibition rate for 28 days was 30%. Accordingly, the compound 1 is useful as a therapeutic agent for prostate cancer.

Example 2 Antitumor Activity Test 2

Male nude mice (age 4-5 weeks) without thymus gland were used in the test. The nude mice were bred in an aseptic condition.
Human prostate cancer LNCaP cells were purchased from the American Type Culture Collection (ATCC). LNCaP cells ($5 \times 10^6$) were subcutaneously transplanted to the nude mice to form a solid tumor and then used in the test.

The lower abdomens of the nude mice were incised about 2 cm under anesthesia to expose the prostate. The prostate membrane was carefully opened, and a LNCaP tumor fragment was inserted inside. The opening in the prostate membrane was closed with absorbable suture. The prostate was returned in the abdominal cavity, and the incision in the lower abdomen was sutured with absorbable suture. These mice were randomly assigned to groups of 20 animals each. These groups were designated as compound 1 group to which compound 1 (20 mg/kg) was administered, Finasteride group to which Finasteride (20 mg/kg) was administered and non-dosed control group.

The transplanted tumor was confirmed to be taken into the mouse when the tumor grew until it could be measured from outside the mouse body, and then administration of compound (1) and Finasteride was started. The compound 1 and Finasteride were orally administered daily at the dose levels described above. The volume of the transplanted tumor was calculated according to the formula shown in Example 1. In addition, the mice were immediately necropsied when they died due to the cancer and the tumor was weighed. The presence of metastasis was also confirmed at the same time. Tissue section of metastasis was prepared by imbedding in paraffin after fixing with 10% formalin. The tissue sections were stained with hematoxylin and eosin followed by examination of the metastasis.

Student's t-test was performed on the transplanted tumor weights for all groups. In addition, Fisher's test was performed on cases of metastasis in each of the groups. The Mann-Whitney U-test and Student's t-test were performed on the two groups used for comparison with respect to life-extending effects. The chi squared ($\chi^2$) test according to Pearson was performed on survival rate on day 63 after tumor transplantation in particular. The hazard rate of less than 5% was regarded as significant for these tests.

Results

[Table 1]

| Experimental Group | Survival Rate (%) |
|---|---|
| Control group | 0 |
| Compound 1 | 56 |
| Finasteride | 22 |

As is clear from Table 1, the compound 1 group exhibited a much better survival rate in comparison with the Finasteride group.

Reference Example 1

N-[1-Methyl-1-(4-methoxyphenyl)ethyl]-3-oxo-4-aza-5α-androst-1-ene-17β-carboxamide

1.0g of 3-oxo-4-aza-5α-androst-1-ene-17β-carboxylic acid, 1.6 g of triphenylphosphine and 1.4 g of 2,2'-dipyridyldisulfide were successively added to 30 ml of dry toluene and the mixture was stirred at room temperature overnight. The reaction mixture as such was subjected to column chromatography on 35 g of silica gel and was eluted with acetone/methylene chloride (1:9 to 1: 1) to obtain 1.11 g of 2-pyiridylthio ester derivative.

5.0 g of 2-pyiridylthio ester derivative synthesized in a similar manner to that described above and 5.0 g of 1-(4-methoxyphenyl)-1-methylethylamine were added successively to 30 ml of dry methylene chloride and the mixture was stirred at room temperature for 3 days. The reaction mixture was diluted with 100 ml of methylene chloride, washed with 1N hydrochloric acid, water, aqueous sodium hydrogencarbonate and a saturated saline solution successively. The methylene chloride layer was dried over magnesium sulfate and then concentrated under reduced pressure. The residue was subjected to column chromatography on 15 g of silica gel and was eluted with acetone/methylene chloride (1:9 to 1:1) to obtain 5.2 g of the title compound.
NMR spectra ($CDCl_3$) δ ppm: 0.68 (3H, s), 0.98 (3H, s), 0.90-2.20 (16H, m), 1.70 (3H, s), 1.72 (3H, s), 3.35 (1H, t, J=9 Hz), 3.80 (3H, s), 5.48 (1H, br.), 5.76 (1H, br.), 5.83 (1H, d, J=10 Hz), 6.82 (1H, d, J=10 Hz), 6.88 (2H, d, J=9 Hz), 7.32 (2H, d, J=9 Hz)
IR spectra $\nu_{max}$ $cm^{-1}$ (KBr): 2969, 2938, 1672, 1599, 1514, 1455, 1248, 1181, 1035, 825

[Industrial Applicability]

The compound used in the present invention has excellent antitumor activity and it is weak in toxicity. Thus, it is useful as a composition for treatment or prevention of prostate cancer.

The compound or the pharmacologically acceptable salts thereof used in the present invention is used as a composition for treatment or prevention of prostate cancer. The compound itself or mixtures of the compound with appropriately pharmacologically acceptable excipients, diluents and the like can be orally administered as tablets, capsules, granules, powders or syrups.

These pharmaceutical preparations are prepared by standard techniques that are well known to those skilled in the art using additives. The additives are excipients (for example, organic excipients such as sugar derivatives, e.g. lactose, sucrose, glucose, mannitol and sorbitol; starch derivatives such as corn starch, potato starch, α-starch, dextrin and carboxymethyl starch; cellulose derivatives such as crystalline cellulose, low substituted hydroxyproyl cellulose, hydroxypropylmethyl cellulose, carboxymethyl cellulose, calcium carboxymethyl cellulose and internally bridged sodium carboxymethyl cellulose; gum Arabic; dextran; and Pullulan; and inorganic excipients such as silicate derivatives, e.g. light silicic acid anhydride, synthetic aluminum silicate and magnesium meta-silicic acid aluminate; phosphates, e.g. calcium phosphate; carbonates, e.g. calcium carbonate; and sulfates, e.g. calcium sulfate); lubricants (for example, stearic acid; metal salts of stearic acid such as calcium stearate and magnesium stearate; talc; colloidal silica; waxes such as beeswax and spermaceti; boric acid; adipic acid; sulfates such as sodium sulfate; glycol; fumaric acid; sodium benzoate; DL leucine; sodium salts of aliphatic acid; laurylsulfates such as sodium laurylsulfate and magnesium laurylsulfate; silicic acids such as silicic acid anhydride and silicic acid hydrate; and the above-mentioned starch derivatives); binders (for example, polyvinyl pyrrolidone, Macrogol and the same compounds as described in the above excipients); disintegrants (for example, the same compounds as described in the above excipients; and chemically modified starches and celluloses such as sodium Crosscarmelose, sodium carboxymethyl starch and bridged polyvinyl pyrrolidone); stabilizers (for example, para-oxybenzoates such as methylparaben and propylparaben; alcohols such as chlorobutanol, benzyl alcohol and phenylethyl alcohol; benzalkonium chloride; phenols such as phenol and cresol; thimerosal; dehydroacetic acid; and sorbic acid); corrigents (for example, sweetening agents, acidifiers and aroma chemicals conventionally used); and diluents.

The dose varies depending on the condition and age of the patient. For example, it is desirable to administer 0.001 mg/kg body weight (preferably 0.01 mg/kg body weight) as a lower limit and 20 mg/kg body weight (preferably 1 mg/kg body weight) as an upper limit once to several times a day depending on the symptoms.

Preparation examples are shown as follows. However, the scope of the invention is not limited to these examples.

| Preparation Example 1 | |
|---|---|
| Capsules | |
| Compound of Reference Example 1 | 20.0 mg |
| Lactose | 158.7 |
| Corn starch | 70.0 |
| Magnesium stearate | 1.3 |
| | 250 mg |

Powders described above were mixed and sieved through 60 mesh sieve and then the resulting powders were encapsulated in a No. 3 gelatin capsule of 250 mg to give a capsule.

| Preparation Example 2 | |
|---|---|
| | Tablets |
| Compound of Reference Example 1 | 20.0 mg |
| Lactose | 154.0 |
| Corn starch | 25.0 |
| Magnesium stearate | 1.0 |
| | 200 mg |

Powders described above were mixed and formed into a tablet using a tablet making machine to obtain a tablet of 200 mg.

The tablet may be coated with sugar, if necessary.

## Claims

1. Use of N-[1-methyl-1-(4-methoxyphenyl)ethyl]-3-oxo-4-aza-5α-androst-1-ene-17β-carboxamide, or a pharmacologically acceptable salt thereof or other derivative thereof for the manufacture of a pharmaceutical preparation for the treatment or prevention of prostate cancer.

2. The use according to claim 1 wherein said pharmaceutical preparation is in a form suitable for oral administration.

## Patentansprüche

1. Verwendung von N-[1-Methyl-1-(4-methoxyphenyl)ethyl]-3-oxo-4-aza-5α-androst-1-en-17β-carboxamid oder eines pharmakologisch akzeptablen Salzes davon oder eines anderen Derivats davon zur Herstellung einer pharmazeutischen Zubereitung zur Behandlung oder Vorbeugung von Prostatakrebs.

2. Verwendung nach Anspruch 1, worin die pharmazeutische Zubereitung in Form einer geeigneten oralen Verabreichung vorliegt.

## Revendications

1. Utilisation de N-[1-méthyl-1-(4-méthoxyphényl)éthyl]-3-oxo-4-aza-5α-androst-1-ène-17β-carboxamide, ou de l'un de ses sels pharmacologiquement acceptables ou d'un autre de ses dérivés, pour la fabrication d'une préparation pharmaceutique pour le traitement ou la prévention du cancer de la prostate.

2. Utilisation selon la revendication 1, dans laquelle ladite préparation pharmaceutique est sous une forme appropriée à l'administration orale.

Fig 1 Antitumor effect against a human prostate cancer